(19) Europäisches Patentamt
European Patent Office
Office européen des brevets

(11) **EP 2 329 765 A1**

(12) **EUROPEAN PATENT APPLICATION**

(43) Date of publication:
**08.06.2011 Bulletin 2011/23**

(51) Int Cl.:
***A61B 5/029*** *(2006.01)*

(21) Application number: **10011456.0**

(22) Date of filing: **26.04.2000**

| | |
|---|---|
| (84) Designated Contracting States:<br>**AT BE CH CY DE DK ES FI FR GB GR IE IT LI LU MC NL PT SE** | (72) Inventor: **Romano, Salvatore**<br>**50144 Firenze (IT)** |
| (30) Priority: **27.04.1999  IT FI990098** | (74) Representative: **Santi, Filippo et al**<br>**Barzano' & Zanardo Roma S.p.A.**<br>**Via Piemonte 26**<br>**00187 Roma (IT)** |
| (62) Document number(s) of the earlier application(s) in accordance with Art. 76 EPC:<br>**00929424.0 / 1 173 093** | <u>Remarks:</u><br>This application was filed on 29-09-2010 as a divisional application to the application mentioned under INID code 62. |
| (71) Applicant: **Romano, Salvatore**<br>**50144 Firenze (IT)** | |

(54) **Method and apparatus for measuring cardiac flow output**

(57)    Method for the measurement of the cardiac flow, in which the volume of blood ejected from the left ventricle (LSV) or the volume of blood ejected from the right ventricle (RSV) is espresse and calculated as a function of at least one of the independent contributing factors of the area under the pressure curve recorded by menas o fan appropriate sensor and as a function of the hydraulic impedance, and in which th cardiac flow Q is calcuated according to the relation Q = LSV (or RSV) *HR, where HR = Heart Rate.

Fig. 1

EP 2 329 765 A1

## Description

### Field of the invention

The present invention refers to a method and an apparatus for determining the stroke volume - i. e., the volume of blood expelled from the left ventricle (LSV), the volume of blood expelled from the right ventricle (RSV) - and hence the cardiac output Q - i. e., the stroke volume multiplied by the heart rate (HR) -, in a continuous way, using invasive and non-invasive indirect techniques, so as to enable acquisition of this important haemodynamic parameter in various clinical and non-clinical situations, as well as in the course of ergometric tests.

### Prior art

For the measurement of cardiac flow Q, the invasive methods that are currently most extensively used are the Thermodilution Method (TDM), Fick's Method (FM), and a method that uses the arterial pressure signal p (t) measured in the aorta or in the pulmonary artery, referred to as the Pulse Contour Method (PCM).

This method which uses the signal pressure is not very reliable and for this reason it is necessary to make a calibration. This is usually the TDM. At the present time reliable results have not been attained by this method.

This PCM method derives from an original idea of Herd [Herd J. A. et al., 1864] and from the theory referred to as the Windkassel (German for"air chamber") theory of Franck (Franck O., 1930), and is based on the existence of a relationship between the volume of blood expelled by the left ventricle (LSV) or the volume of blood expelled by the right ventricle (RSV), and the area under the pressure curve p (t).

The fundamental relation used for calculating the stroke volume is $SV = A/Z0$ where A, expressed in [mmHg t], is the area under the pressure curve p (t) (see figure A1), and Z0, expressed in [mmHg/cm/t], is the hydraulic impedance which depends upon the dynamic resistances and upon the compliance of the artery wall. LSV is measured in [cm3] (see figure A1), hence $Q = LSV*HR$ is the cardiac flow expressed in litres per minute if the heart rate is measured in beats per minute. In this connection we recall that the plot of the arterial pressure with respect to time is determined by the magnitude of LSV and by the vascular impedance. Consequently, the Pulse Contour Method endeavours to separate and analyse these two contributions; however, the method is unable to determine the two contributions as independent variables over time.

Applying the Windkassel theory, many studies have attempted to determine LSV only from the pressure waveform and from the characteristics linked to transmission of the wave in the aorta or in the pulmonary artery [Remington J. W. et al., 1948; Warner H. R. et al., 1953; Herd J. A. et al., 1966; Kouchoukos N. T. et al., 1970].

The original idea of Franck has subsequently been applied over the years and has made it possible to estimate LSV in a continuous way from the measurement of the pressure signal in the aorta or in the pulmonary artery [McDonald D. A. et al., 1974; Wesseling K. H. et al., 1976; Tajimi T. et al., 1983; Wesseling K. H. et al., 1993].

However, in the concrete application to the various possible clinical situations, the Pulse Contour Method currently requires a"calibration"for calculation of the hydraulic impedance. For the calibration, generally one of the other two methods referred to above, i. e., the thermodilution method and Fick's method, is used, or else linear regressions of aortic parameters, such as the diameter of the aorta, and the age, sex, height and weight of the patient are used.

Unfortunately, the calibration and regression factors are subject to error, given that the methods from which they are obtained are in turn imprecise and that the regressions are in any case obtained on a limited number of subjects, and are hence acceptable only as a mean and not as a true measurement of the quantity investigated.

In fact, cardiac flow estimated using the thermodilution method and Fick's method are not always in agreement with the clinical parameters obtained using other diagnostic techniques, and this mainly occurs in patients suffering from certain forms of heart disease, such as disease involving dilatation of the heart, valvular cardiopathy, and cardiac fibrillation.

To provide an example, consider two possible signals in the aorta studied between the points of opening and closing of the ventricle. These signals will in general present the same area but different forms, with different times of attainment of the systolic point.

The traditional Pulse Contour Method will therefore yield the same exact measurement (same integral) evaluated on the basis of the calibration impedance.

However, it is evident that from a different form of the signal there must derive a different impedance, which cannot be evaluated.

Hence, the limits of the invasive techniques currently in use are: a) the poor precision achievable in the estimation of cardiac flow on account of clinical illnesses; b) the non-applicability in general on account of the pathological condition of the patient; and c) the impossibility of applying the said invasive techniques, for example during an ergometric test.

**Scope of the invention**

A first scope of the invention is to obtain measurements in a continuous way that are more reliable than the ones obtained using the invasive and non-invasive techniques currently applied.

A second scope is to render the measurement substantially independent of the point of application of the sensor by introducing variations in the specific formulas, without any need for prior calibration of the measurement.

**Summary of the invention**

The above purposes have been achieved according to the invention using a method which directly obtain the cardiac flow from the pressure signal measured in an invasive way, in the ascending aorta, in the pulmonary artery and in femoral brachial and radial, or measured in a non-invasive way, for example from the arteriole of the finger using a cuff meter. According to the method, the impedance of the pressure signal is calculated on the basis of the points of resonance of the signal by assimilating the signal to that of a flow in an elastic tube and assuming constancy of Young's modulus, which is in particular taken to have a value of unity. In this way, it is possible to calculate the cardiac flow without any longer having to use various calibrations, but exclusively from an analysis of the pressure wave and its characteristics.

Preferably, the hydraulic impedance is calculated by means of an analysis of the first and second derivatives with respect to time of the pressure signal recorded.

According to a further aspect of the invention, a correction is moreover made of the mean pressure value to be used for the calculation of LSV in order to take into account the attenuations of the said value in the various points of possible recording of the signal.

According to a further aspect of the invention, from the signal recorded in the finger (or from some other point in a non-invasive way), the method makes possible a direct reconstruction of the signal in the aorta and in the pulmonary artery, and from the latter signal a reconstruction of the cardiac flow.

More specifically, according to the invention In order to obtain the SV estimation we have taken into consideration the wave pressure in the ascending aorta and/or in pulmonary artery, the artery compliance (E) and the periferical resistance (R).

Therefore we have taken into consideration that 1) the SV depends on the pressure variation obtained at the opening of the ventricular valve (that is the difference between systolic and diastolic pressure divided by the time necessary to pass between the systolic and diastolic) and 2) the SV is conditioned by E and R.

In order to obtain these contributions we have taken into account the value of the dicrotic pressure and the other characteristic points between the systolic dicrotic pressure (this pressure values must be divided by time. This time is the difference between the end of the cardiac beat and the time of the event being taken into consideration).

Therefore we have considered SV as being determined by three points: 1) the bolus of blood ejected by the ventricular; 2) the reaction due to the aortic wall; 3) resistance due to periferic arterial cycle. As the value of the pressure at the point where it is taken is the result of these three components at the same time we have studied our system in a perturbative way. Therefore we have considered the principle contribution of the ventricular and of the system E and R, the first being given by 1) as described above and the second, the E and R system principally contributes to the closure of the valve (point of diacrotic pressure). This last event point is conditioned by a series of perturbations on the pressure signal after the cardiac valve, according to the vase being crossed and the length of the course.

That is it is necessary to take into consideration not only the contribution due to the systolic and to the diacrotic above described, and when present those due to secondary perturbations.

In conclusion all the event points which have been taken into consideration are the moments in which there is a state of balance between the various points (blood ejected from the ventricular-E-R): the "principle" balancing points (systolic and dicrotic points) can be or not "accompanied" by other balancing points (how and if to analyse them will be described later). All this information can be found in the wave pressure which flows after having been generated by the ventricular (right or left).

Advantageously, with the method according to the invention it is possible to establish a relationship between the hydraulic impedance and the usable time, also in combination with known methods (e. g., the thermodilution method) which involve a phase of calibration of the signal recorded, where the contribution of the area under the pressure curve is considered variable over time, whilst the contribution of the impedance can only be considered as constant.

In particular, by the method of the present invention (hereinafter called pulse analytical method (PAM)) it has been possible: a) to find the SV from the signal pressure recorded invasively in ascending aorta and in pulmonary artery; b) to find the SV from the arterial signal pressure invasively recorded (brachial, radial, and femoral artery) and non invasively recorded (e. g. from the pressure obtained with the oscillometric method from the arterial finger).

In this way we estimated the LSV and RSV and so we determined the true Q in a way that is completely free from any calibration. Therefore these results are obtained only by the analysis of the wave pressure (depending only on where the wave pressure was taken).

According to the invention an apparatus able to perform the method is provided.

The apparatus comprises a microprocessor unit able to receive the blood pressure signal and to analyse it over the time in order to determine the above identified parameters and to calculate the cardiac output Q therefrom.

In a preferred embodiment the apparatus further comprises a sensor in the shape of a cuff meter able to be applied to a finger and to acquire the blood pressure signal.

**Brief description of the figures**

- Figure A1 shows a cardiac pressure signal diagram as analysed by the prior art method;
- Figures 1-19 show the form of the cardiac pressure signal and of its first and second derivatives, the signal being taken in various recording points;
- Figure 20 shows a reconstruction, according to the present method, of the signal in the aorta starting from the pressure signal recorded at the arteriole of the finger.

**Detailed description of the invention**

In the following, pressure is expressed in the unit "mmHg", which has the following conversion relationship with the appropriate SI unit "Pascal":

$$1 \text{ mmHg} = (101325/760) \text{ Pascal.}$$

With reference to the annexed figures, various examples of application of the method are described in what follows.

Example I

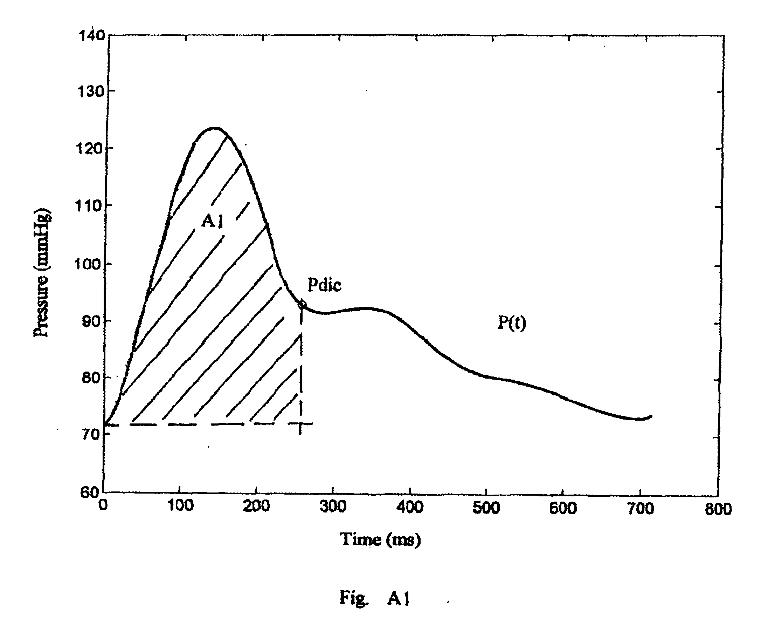

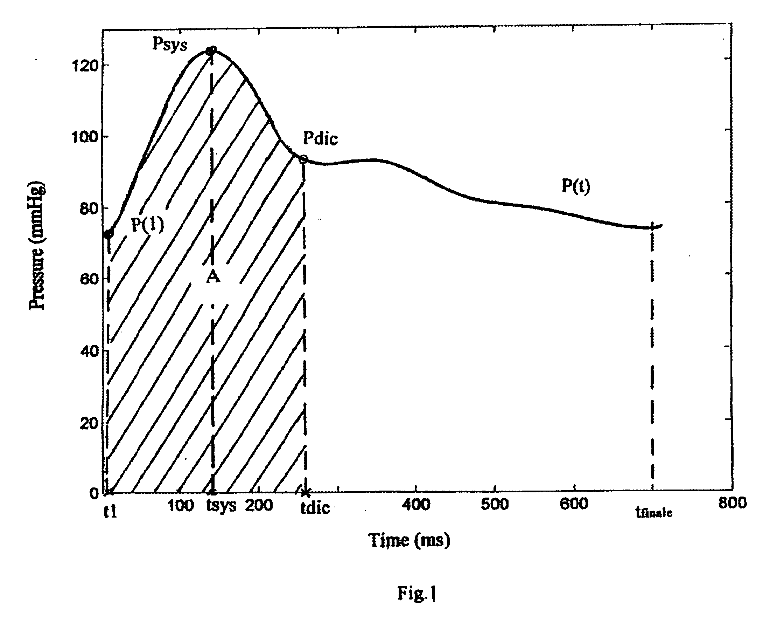

A) Relation between LSV and pressure taken in the ascending aorta (Pulse Analytical Method Aortic: PAMA) (Fig. 1-6)

i) The PAMA determines the heart flow Q in litres per minute using the following general relation (the pressure signal is acquired in the ascending aorta at 1000 Hz):

$$\text{LSV} = [K[A/((Za1+Za2) *1000)+A/((Za1+Za2)*1000)*(Pm-K1)/K1]]/1000$$

$$\text{Eq. [1]}$$

where:

K = 1 and has the dimensions $[(\lambda m*sqrt(2p/(p)*Vm], \text{ expressed in } [l^3/t^2]; .$
$\lambda m$ is the mean wavelength, approximately equal to 10 m
Vm is the mean velocity, approximately equal to 10 m/s
p is the blood density;
A is the integral between t1 (time at the diastolic pulse in [ms]) and tdic (time at the dicrotic pressure in [ms]) under the pressure curve p(t), expressed in [mmHg*ms] (Figure 1);
K1 = 100, expressed in [mmHg], and represents the correction factor of the mean pressure;
Za1 = (psys-p(1))/tsys, expressed in [mmHg/ms];
Za2 = (pdic/tfinal-tdic), expressed in [mmHg/ms]; and
Pm = (psys+2p(1))/3. see the following Note 1
ffinal= time of the beat being taken into consideration (time origin in t1 and final in tfinal)

The cardiac flow is thus

$$Q = LSV*HR$$

where Q is expressed in [lit/min];
HR = 60000/T; and
T is the cardiac period expressed in [ms].

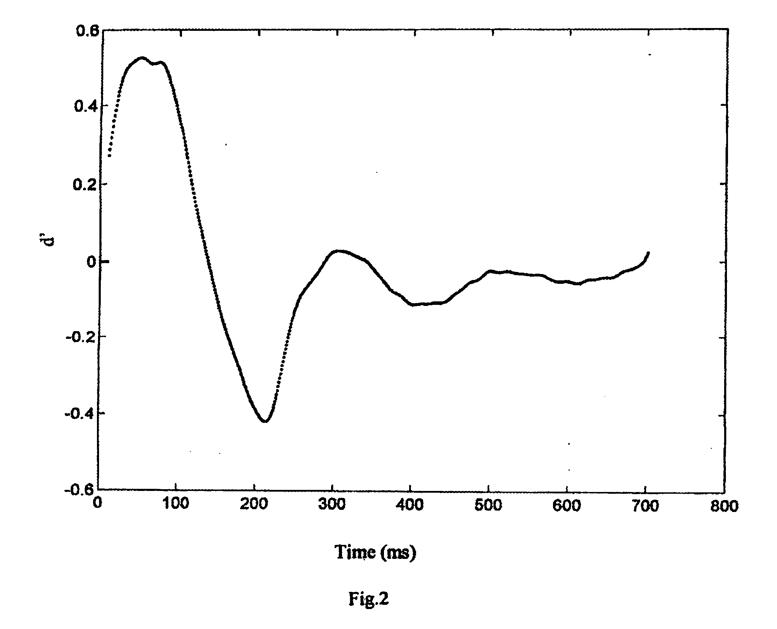

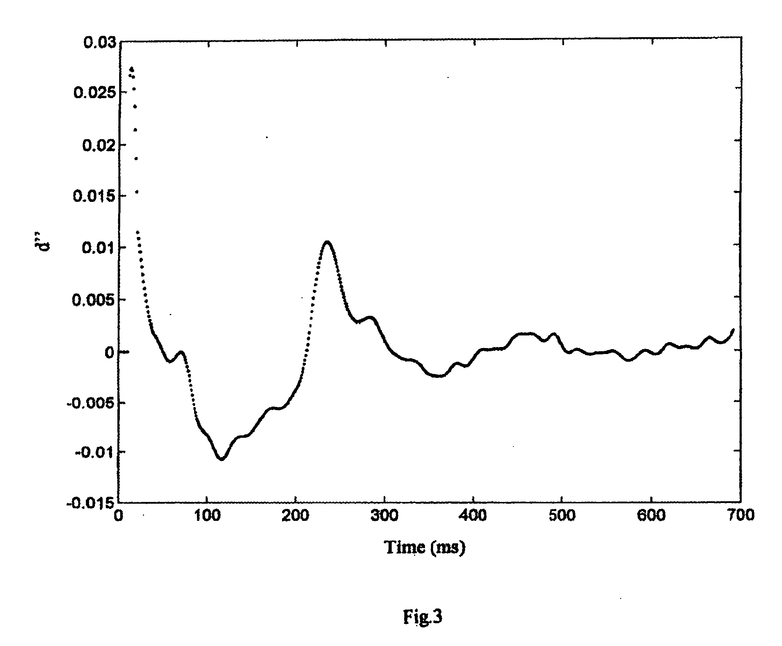

This relation was applied in cases in which the pressure curve and the corresponding mean of the tangent at 21 points (i. e., the first derivative d') and the mean of the tangent at 21 points of the mean tangent (i. e., the second derivative d") were those shown in Figures 2 and 3 and could be associated to the recording point.

ii) With-Za3

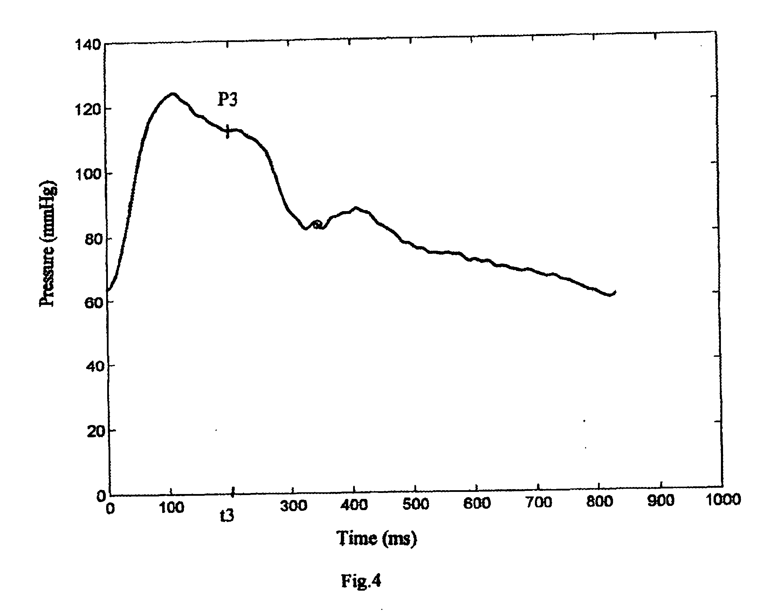

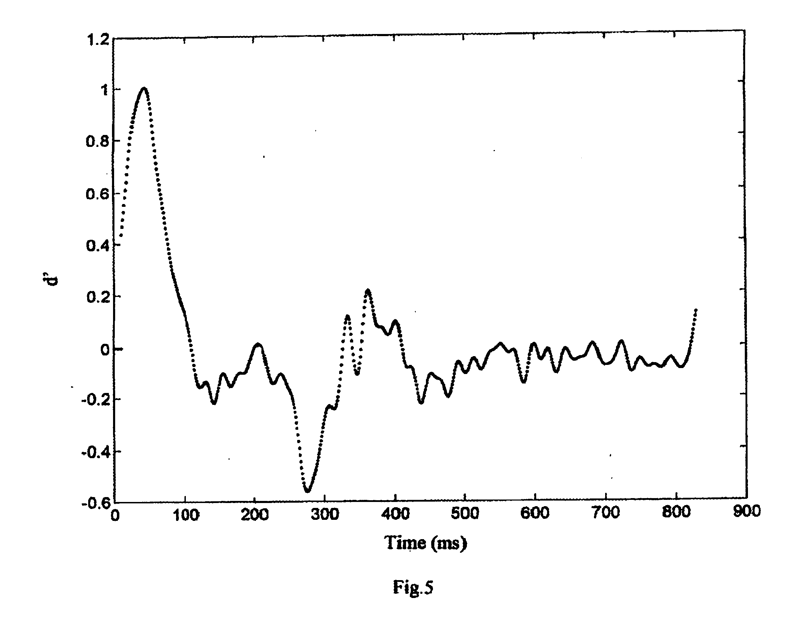

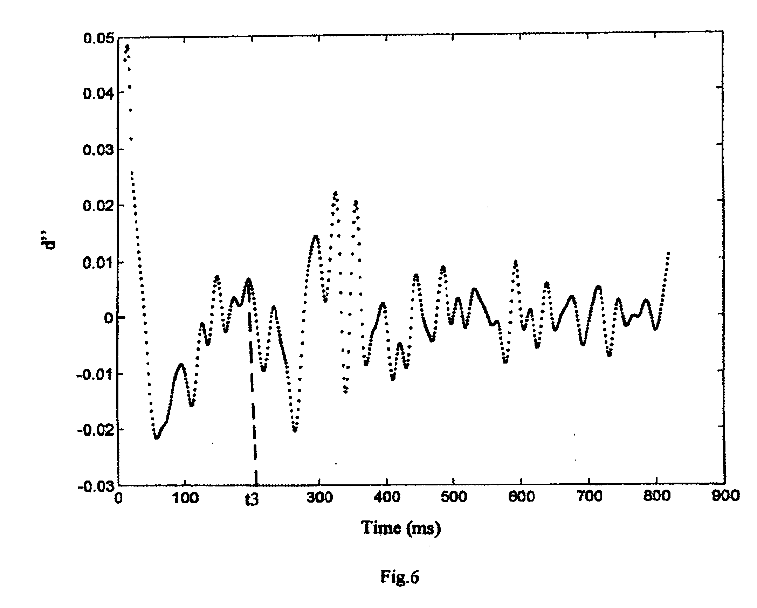

In the cases where the pressure curves in the ascending aorta were of the type shown in Figure 4, and the corresponding first and second derivatives, d' and d", were as those shown in Figures 5 and 6 and revealed the point of resonance at time t3, then the relation became:

$$LSV=[K[A/((Za1+Za2-Za3)*1000)+A/((Za1+Za2-Za3)*1000)*(Pm-K1)/K1]]/1000$$

$$Eq. [2]$$

where the symbols have the same meaning as in Eq. [1], and where t3 is the time, in [ms], at the minimum value of d"between the time tsys and the time tdic, and p3 is the corresponding pressure expressed in [mmHg] at time t3 (see Figure 6) and

$$Za3 = (P3/ (tfinal-t3) \qquad mmHg/ms$$

In a similar way it is possible to calculate Q = LSV*HR.

Note 1

The mean pressure for the pressure measured in the ascending aorta must be considered as such for the interval 90-110 mmHg; for the mean pressure between 110-120 and 90-80 mmHg it must be considered at 50% (for example for a Pm= 118 mmHg for our method is = 114 mmHg); for mean pressure values between 120-130 and 80-70 mmHg it must be considered at 25%, for mean pressure values >=130 and <=70 mmHg it must be considered 13%.

Example II

B) Relation between RSV and the pressure taken in the pulmonary artery (Pulse Analytical Method, Pulmonary : PAMP)

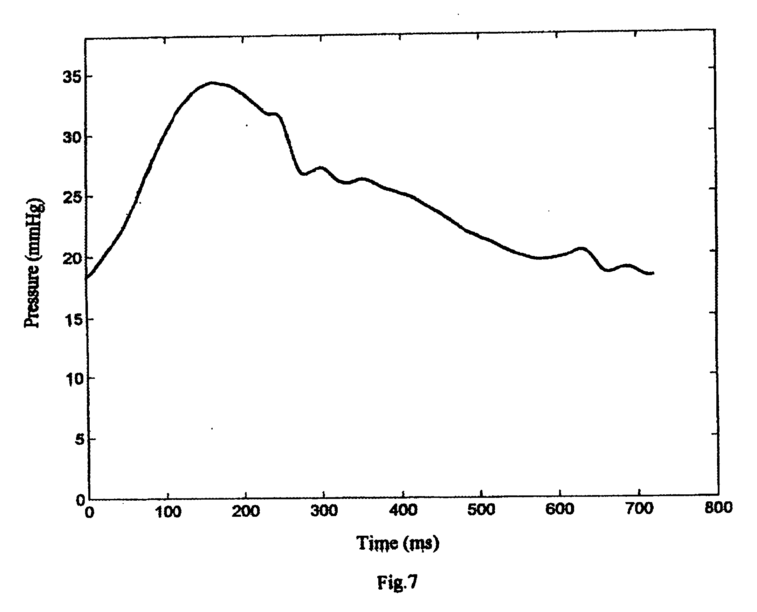

Relationship between the volume of blood expelled from the right ventricle RSV and the pressure measured in the pulmonary artery. The corresponding signal pressure is similar to the one represented for aortic pressure, but for variations in scale (see Figure 7).
The PAMP determines the heart flow Q in litres per minute using the following general relation (the pressure is acquired in the pulmonary artery at 1000 Hz):

i) Case with mean pressure in pulmonary artery >= 19mmHg

$$RSV=[K[A/((Za1+Za2)*1000)+A/((Za1+Za2)*1000)*(Pm-K1)/K1]]/1000$$

$$[Eq. 3]$$

where:

K = 1 and has the dimensions [(λm*sqrt (2p/(p)*Vm], expressed in [$l^3/t^2$], p being the density of the blood;
A is the integral between t1 (time at the diastolic pulse in [ms]) and tdic (time at the second dilatation of the artery in a dicrotic pulse in [ms]) under the pressure curve p (t), expressed in [mmHg*ms];
K1 = 12, expressed in [mmHg];
Za1 = (psys)/tsys, expressed in [mmHg/ms];
Za2 = (pdic/tfinal-tdic), expressed in [mmHg/ms] ; and
Pm = (psys+2p(1)/3);          see following Note 2
Q = RSV*HR,
where Q is expressed in [lit/min];
HR = 60000/T; and
T is the cardiac period expressed in [ms].

In Figure 7 a signal acquisition of the pressure in the pulmonary artery is shown.
For the pressure in pulmonary artery we have variations for d' and d"similar to those of the aorta. In consequence the determination of the point of dicrotic pressure (Pdic), the systolic pressure (Psys), diastolic pressure (P (1)) and the relative times is as described above.
ii) Case with mean pressure in pulmonary artery <= 19mmHg
In the cases in which Pm is <= 19 mmHg the relation becomes: RSV=[K[A/((Za1+Za2)*1000)+A/((Za1+Za2)*1000]] /1000 **Eq. [4]** with the same meaning for the symbols as in the preceding cases. In the same way it is possible to calculate Q=RSV*HR

Note 2

The mean pressure in the case of pressure taken in the pulmonart artery must be taken as such for the interval of pressure between 19-28 mmHg; for values of mean pressure between 28-33 mmHg it must be considered at 50%, for values of mean pressure > 33mmHg it must be considered at 25% (for example a pm=43mmHg for our method is equal to 33 mmHg); for values < 19mmHg we are in case ii) and therefore we do not use the mean pressure.

Example III

C) Relation between LSV and the pressure non invasively recorded from the arterial finger (Pulse Analvsis Method. Finger: PAMF)

Direct relationship

i) The PAMF determines the cardiac flow Q in litres per minute using the following general relation (the pressure is acquired at the finger of the left hand at 1000 Hz):

$$LSV = [k[A/((Zf1+Zf2)*1000)+A/((Zf1+Zf2)*1000)*(Pm-K1)/K1]]/1000$$

$$Eq. [5]$$

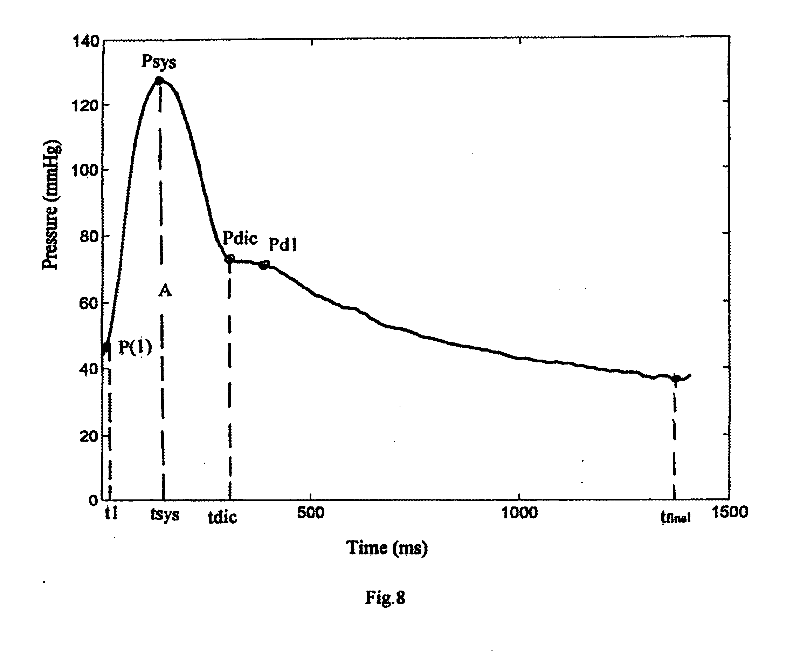

where (Figure 8):

K = 1 and has the dimensions [(λm*sqrt (2p/(p)*Vm], expressed in [$l^3/t^2$];
A is the integral between t1 (time at the diastolic pulse in [ms]) and tdic (time at the dicrotic pressure in [ms]) under the pressure curve p(t),
expressed in [mmHg*ms];
K1 = 90, expressed in [mmHg];
Zf1 = (psys-p(1))/tsys, expressed in [mmHg/ms];
Zf2 = pdic/ (tfinal-tdic), expressed in [mmHg/ms]; and
Pm = (psys+2p (1))/3.          see following Note 3

The corrected volume of blood expelled from the left ventricle (LSVC) is

$$LSVC = [LSV+LSV*abs(delta\ (Pd1-pdic))/(psys-pdias)] \qquad [6]$$

where:

$(Pd1-pdic)$ = variation of pressure of the dicrotic point (Pdic) at its maximum (Pm1) = [mmHg]. This correction exists only when there is an increase in the pressure after the dicrotic pressure $((Pd1-Pdic)>0)$. In the cases in which the increase in pressure is not present $((Pd1-Pdic)<=0)$ we have LSV=LSVC.

Psys is the systolic pressure, expressed in [mmHg];
Pdias is the diastolic pressure, expressed in [mmHg]; and
the term Pd1 is calculated immediately after the dicrotic point and is the maximum value of the curve after (Pdic).

$$Q = LSVC*HR$$

where Q is expressed in [lit/min];
HR = 60000/T; and
T is the cardiac period expressed in [ms].

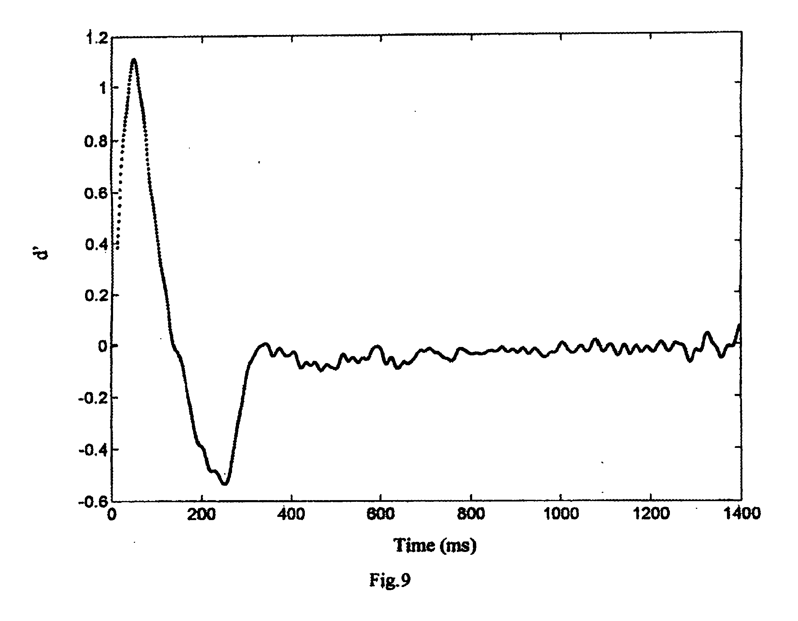

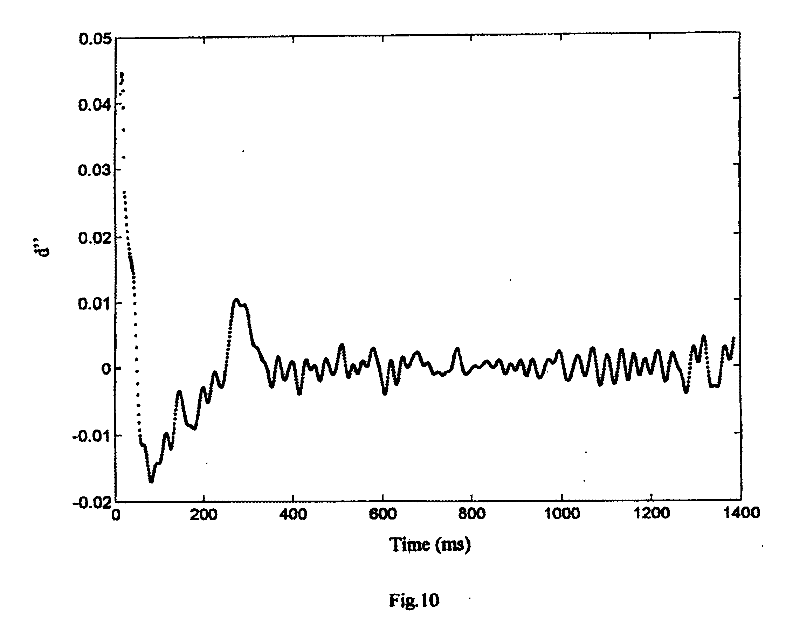

The above relation was applied in the cases where the pressure curve and the corresponding first and second derivatives, d' and d", were those shown in Figures 9 and 10.

ii) With -Zf3

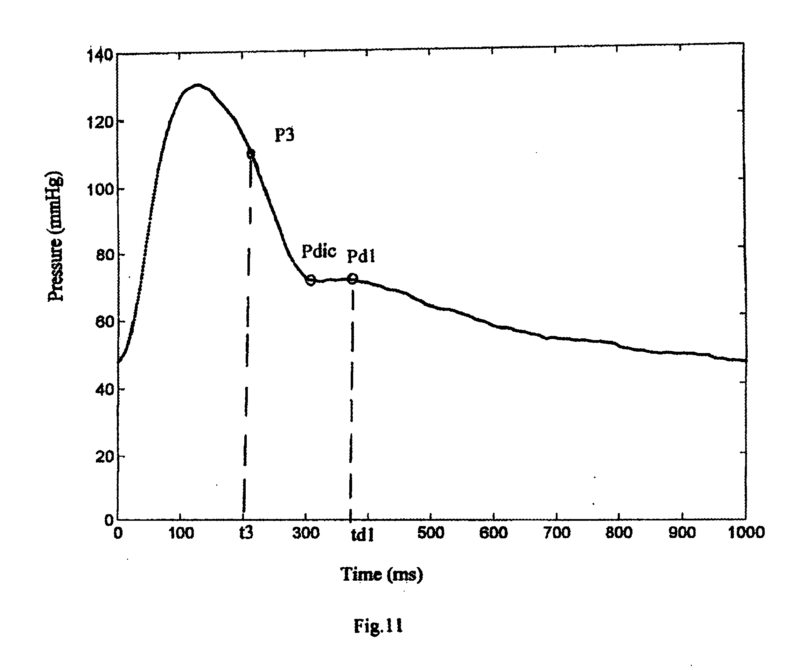

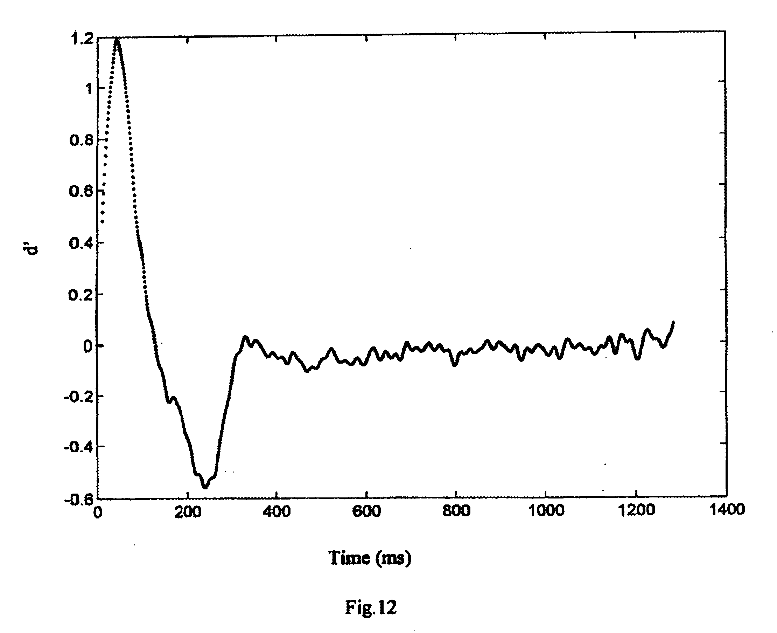

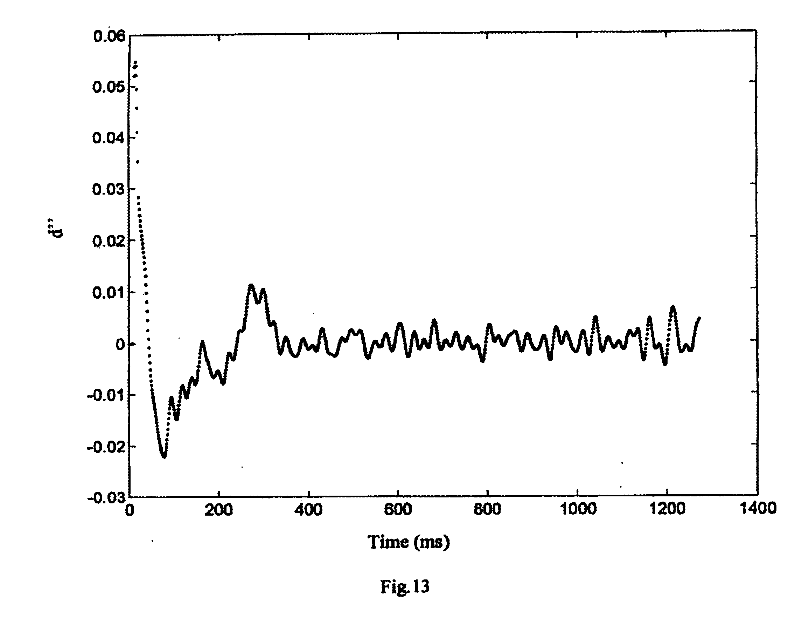

In the cases where the pressure curves were of the type shown in Figure 11 and the corresponding first and second derivatives, d' and d", were as those shown in Figures 12 and 13, the relation became:

$$LSV=[k[A/((Zfl+Zf2-Zf3)*1000)+A/((Zf1+Zf2-Zf3)*1000)*(Pm-K1)/K1]]/1000$$

$$Eq.\ [7]$$

where:

Zf3 = P3/(tfinal-t3); and
the symbols have the same meanings as specified previously, and t3 is the time, in [ms], of the minimum value of d" between the time tsys and the time tdic, and P3 is the corresponding pressure, expressed in [mmHg] at time t3 (see Figure 11).

$$LSVC = LSV+LSV*abs\ (\ (Pd1-Pdic))/\ (psys-P(1)) \qquad Eq.\ [8]$$

In a similar way it is possible to calculate Q = LSVC*HR.

iii) With -2Zf3

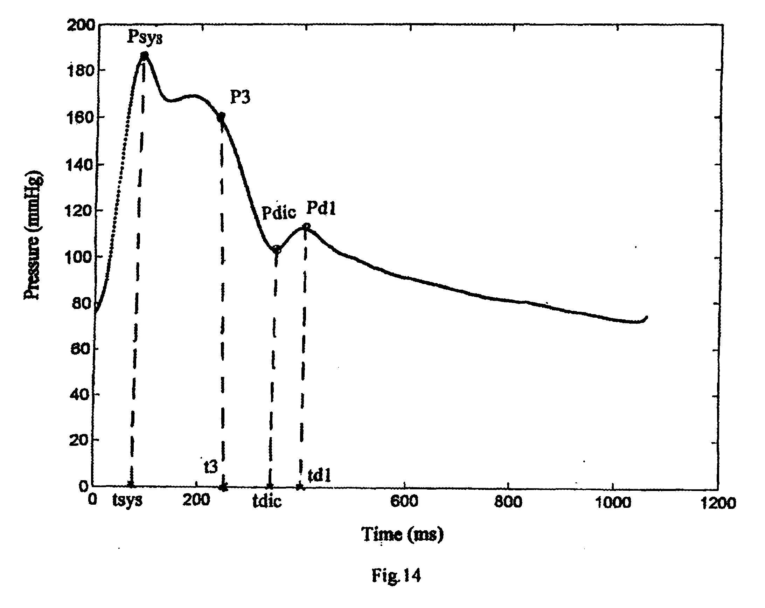

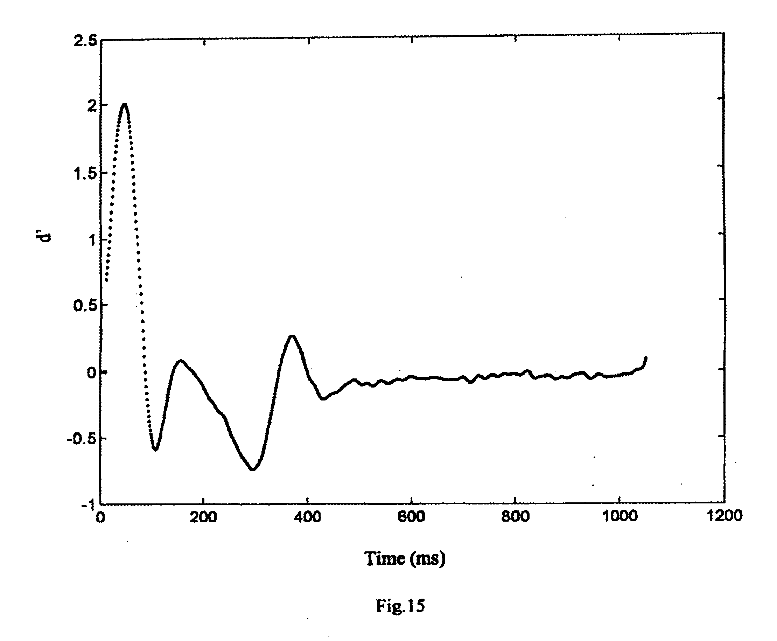

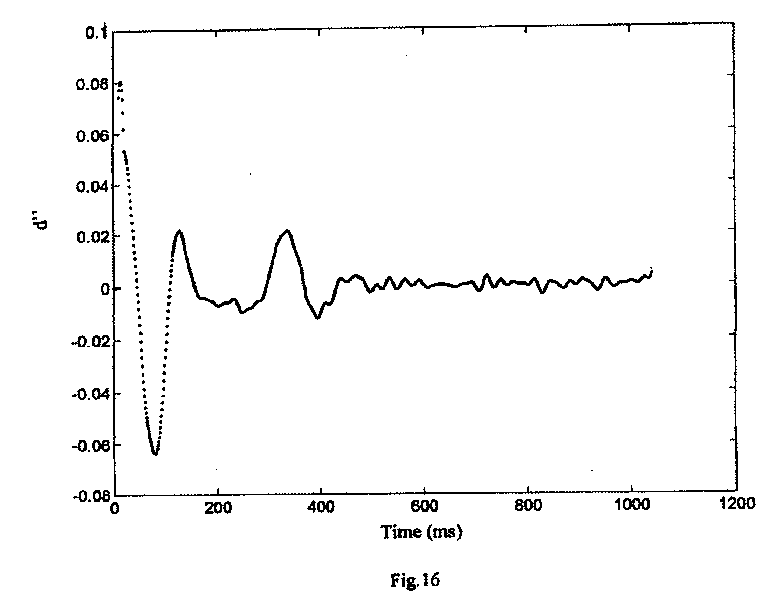

In the cases where the pressure curves were of the type shown in Figure 14 and the corresponding first and second derivatives, d' and d", were as those shown in Figures 15 and 16, the relation became:

$$LSV=[k[A/((Zfl+Zf2-2Zf3)*1000)+A/((Zfl+Zf2-2Zf3)*1000)*(Pm-K1)/K1]]/1000$$

$$Eq.\ [9]$$

where Zf3 = P3/ (tfinal-t3); and
the symbols have the same meanings as specified previously, and t3 is the time, expressed in [ms], of the minimum of d" between the time tsys and the time tdic, and P3 is the corresponding pressure, expressed in [mmHg] at time t3 (see Figure 14).

$$LSVC = LSV + LSV^*abs\ ((Pd1-Pdic))/(Psys-P1) \qquad [10]$$

In a similar way it is possible to calculate Q = LSVC*HR, expressed in litres per minute.

iv) With -2Zf3-Zf5

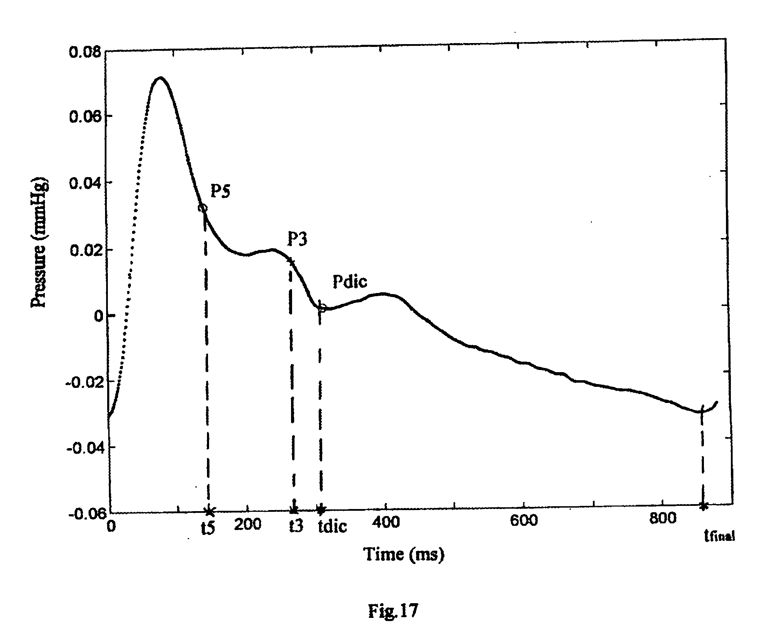

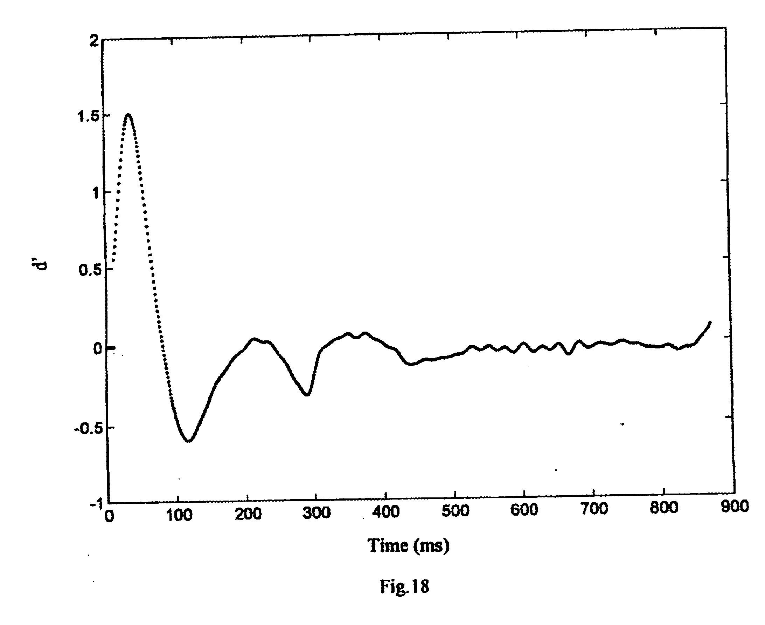

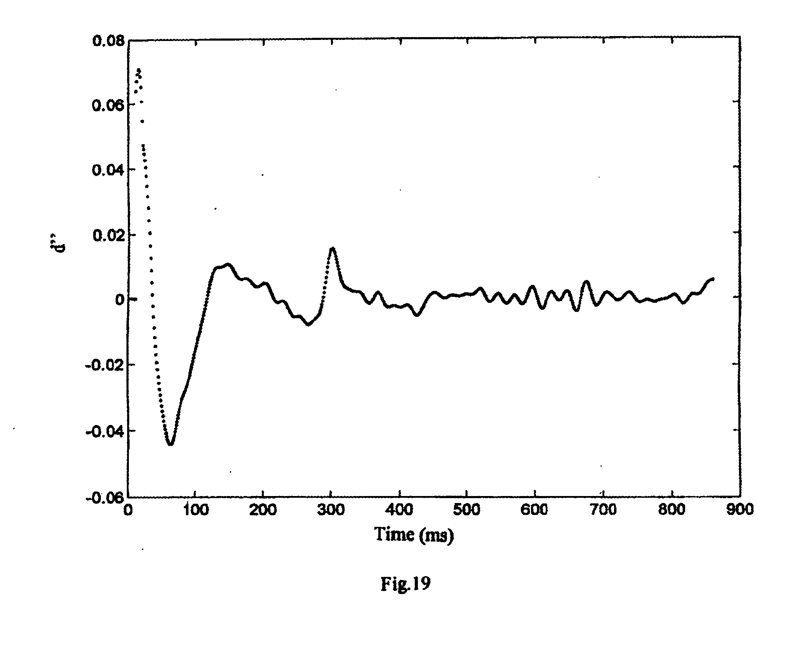

In the cases where the pressure curves were of the type shown in Figure 17 and the corresponding first and second derivatives, d' and d", were as those shown in Figures 18 and 19, the relation became:

$$LSV = [k\ [A/((Zf1+Zf2-2Zf3-Zf5)^*1000)+A/((Zf1+Zf2-2Zf3-Zf5)^*1000)^*(Pm-K1)/K1]]/1000$$

where Zf3 = P3/ (tfinal-t3)

where Zf5 = P5/(tfinale-t5)

the symbols have the same meanings as specified previously, and t5 is the time, expressed in [ms], of the minimum of d" between the time tsys and the time tdic, and P5 is the corresponding pressure, expressed in [mmHg] at time t5 (see Figure 17).

$$LSVC = LSV + LSV^*abs\ ((P1-Pdic))/(Psys-P1) \qquad [11a]$$

In a similar way it is possible to calculate Q = LSVC*HR, expressed in litres per minute.

Note 3

The mean pressure in the case of the pressure taken at the arterial finger non invasively must be considered as such for the interval of mean pressure between 70-110, for the values of mean pressure between 110-150 and 70-40 mmHg it must be considered at 50% (for example a pm=128 for our method is = 119 mmHg); for mean values of pressure > 150 and < 40 mmHg it must be considered at 25%.

v) Reconstruction of the pressure signal in the ascending aorta by means of linear multiple regression in the time domain, by use of Zf1-Zf5

For these reconstruction, basically linear multiple regressions were used. In order to reconstruct the signal recorded in the ascending aorta (or in the pulmonary artery) using a cardiac catheter from the arterial signal recorded in a continuous way by means of a small cuff wrapped around the middle finger of the left hand, a linear multiple regression was used in which the reconstructed pressure signal was obtained in two successive steps:

1) An estimate was made of the mean pressure during the cardiac cycle in the ascending aorta (or in the pulmonary artery) from the signal taken at the finger, deriving the value Pmf (mean pressure in the aorta estimated from the recording taken at the finger) from the formulas used in the various cases of analysis of the arterial signal referred to in the previous points:

$$Pmf = LSV^*Ztot/ (k^*A) \qquad [11b]$$

2) The waveform in the ascending aorta (or in the pulmonary artery) was reconstructed from a fit that used the following parameters:

$$y = a0^*Pmf + a1^*fin + a2^*abs(derfin) + a3^*abs(der2fin) + a4^*abs(der3fin) +$$
$$a5^* (intfin) + a6^*slope^*abs(derfin) + a7^*slope^*zZf1 + a8^*slope$$
$$+ a9^*maxfin + a10^*minfin + a11^*HR^* (intfin(up\ to\ the\ point\ considered))+$$
$$a12^*areaf + a13^*zZf1 + a14^*zZf2 + a15^*zz3f + a16^*zz4f\ a17^*Zf5$$
$$areaf = cof^*(Zf1 +Zf2) / (Zf1+Zf2-n^*z3f- Zf5) \quad [12]$$

where

Zf5 and n = 0, 1 and 2 according to the criteria described previously;
zz4f = Pd1/ (tfinale-td1) (Figure 14);

- fin is the pressure at the finger;
- abs(derfin) is the absolute value of the first derivative in the pressure point considered;
- abs(der2fin) is the absolute value of the second derivative in the pressure point considered;
- abs(der3fin) is the absolute value of the third derivative in the pressure point considered;
- infin is the integral up to the point considered of the signal at the finger;
- slope is the angle between the horizontal axis and the straight line passing through the minimum points on the left and on the right of the cardiac cycle;
- maxfin and minfin coincide with the systolic pressure and the diastolic pressure;
- areaf is the total area of the pressure signal;

and the remaining symbols have the same meanings as specified previously.

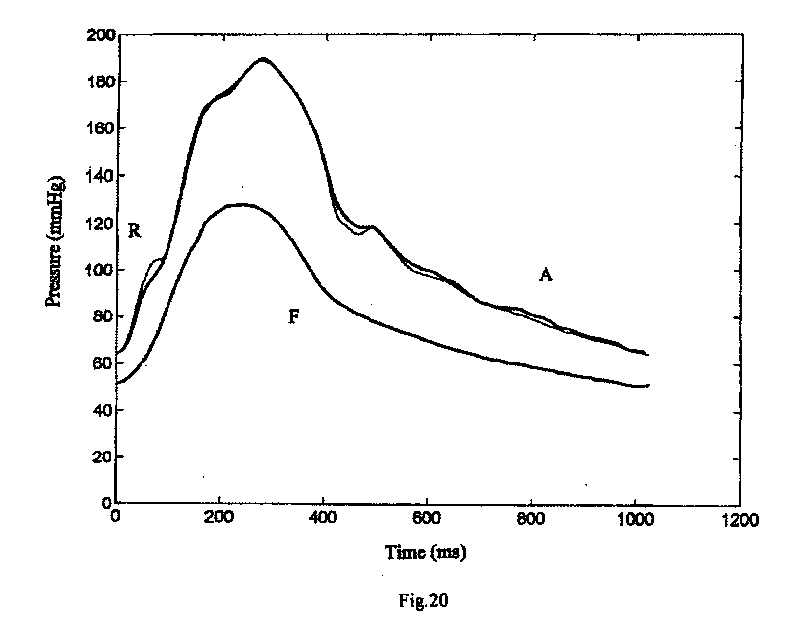

A number of reconstructions obtained are illustrated in Figure 20.
The errors in the comparison between the reconstructed curve of the signal registered non invasively and that taken directly near the ascending aortic are

|  | SD (mmHg) | Max (mmHg) | Min (mmHg) |
|---|---|---|---|
|  | 1.16÷5.67 | 2.38÷16.40 | -2.82 ÷ -16.41 |
| mean: | 3.41 | 9.37 | -9.32 |

with SD= Standard Deviation: the minimum of the interval is obtained for the riconstrruction of the points around the diastolic pressure, the maximum of the difference is obtained near the point of the systolic pressure.
Max= interval of over estimation of the pressure in the point taken into consideration reconstructed and that actually measured with the catheter during the cardiac beat: the minimum of this interval is obtained for the reconstruction of the points around the diastolic pressure, the maximum of the difference is obtained near the points of systolic pressure.
Min= interval of under estimation of the pressure in the point taken into consideration reconstructed and that actually measured with the catheter during the cardiac beat: the minimum of this interval is obtained for the reconstruction of the points around the diastolic pressure, the maximum of the difference is obtained near the points of systolic pressure.
What is important in this calculation are Zf1, Zf2, Zf3, Zf5. which we considered in point C): these are necessary to have satisfactory results.

D) Relation between LSV and the pressure recorded invasively from femoral artery or from another periferical point such as brachial or radial artery (Pulse Analytical Method, Brachial, Radial and Femoral, PAM (BRF))....

For these case we have seen that the formula to use are of the type used in the case C) non invasively with the following precisions: i) K1 for these invasive signals must be considered =100; ii) note 3 remains unchanged.
According to the invention, the method can be applied in combination with known method (such as thermodilution method) comprising a phase of calibration of the signal recorded, in which the contribution of the area under the pressure curve is considered variable over time, whereas the contribution of the impedance can only be considered constant.
In this case, the proposed method also makes it possible to take into account even major variations in the heart rate, in the pressure values and in the pressure waveform for purposes of calculation of the impedance.

It may therefore be concluded that, both in the case of normal subjects and in the case of patients affected by various pathological conditions, the method proposed represents an effective and advantageous diagnostic tool in the invasive and noninvasive evaluation of cardiac flow.

In addition, the method can be applied both in healthy subjects and in subjects presenting cardiocirculatory alterations who are undergoing ergometric tests that are aimed at establishing the level of haemodynamic response to the tests.

It should be emphasized that the present method is based only on the study of the pressure signal (recorded invasively in the pulmonary artery and in the aortic arch, or in any other major arterial vessel, or else non-invasively at the finger), and is independent of the anthropometric data and of the age of the subject examined.

The present invention further comprises an apparatus for measuring cardiac flow, comprising at least one sensor for detecting a blood pressure signal and a computer unit connected to the said sensor for the execution of a measurement according to the above described method and provided with at least one output device of the measured value.

Preferably, the apparatus comprises a storage medium containing a computer program to execute a method according to at least one of the attached method claims.

The invention further relates to a computer program loadable in a computer unit in order to execute the method.

**Claims**

1. Method for measuring cardiac output (CO) of a patient, comprising the following steps: sensing arterial blood pressure and converting the sensed arterial blood pressure to a pressure signal; calculating an estimate of stroke volume as a function only of selected characteristics of the sensed pressure signal, including calculating an area (A) under the entire pressure signal including both pulsatile and non-pulsatile portions of the pressure signal; and calculating an estimate of CO as a function of the estimated stroke volume and a current heart rate value.

2. Method according to claim 1, in which the step of calculating the estimate of stroke volume comprises the following sub-steps: estimating selected impedance values from the pressure signal; and calculating the estimate of stroke volume as a function of a ratio between the calculated area and the estimated selected impedance values.

3. Method according to claim 1 or 2, in which the step of calculating the estimate of stroke volume further comprises the following sub-steps: calculating a mean pressure value of the pressure signal; and correcting the estimated stroke volume as a predetermined function of the mean pressure value and of a reference pressure.

4. Method according to any one of the preceding claims, further including the following steps: detecting times and corresponding pressure values of a systolic peak and of a dicrotic notch in the pressure signal; evaluating a second derivative of the pressure signal between the systolic peak and the dicrotic notch; detecting the time and corresponding pressure value of at least one intermediate point in the pressure signal between the systolic peak and the dicrotic notch at which the second derivative has an extreme value; and estimating at least one of the selected impedance values as a predetermined function of the time and corresponding pressure value of the intermediate poin.

5. Method according to any one of the preceding claims, further including the following steps: detecting a systolic peak pressure Psys, a diastolic pressure Pdia, and a dicrotic pressure Pdic; and scaling the estimated stroke volume by a factor proportional to the ratio between the difference between Pdia and Pdic and the difference between Psys and Pdia.

6. Method according to any one of the preceding claims, further including the following steps: detecting a dicrotic time in the pressure signal; evaluating a post-dicrotic first derivative of a post-dicrotic portion of the pressure signal at times after the dicrotic time; detecting the time and corresponding pressure value of at least one local maximum pressure in the post-dicrotic portion of the pressure signal; and estimating at least one of the selected impedance values as a predetermined function of the time and corresponding pressure value of the local maximum pressure.

7. Method according to any one of the preceding claims, in which the step of calculating the CO estimate is performed based on the pressure signal during a single cardiac cycle.

8. Method according to any one of the preceding claims, in which the pressure signal is uncalibrated, whereby the steps of calculating the estimate of the stroke volume and calculating the estimate of CO are independent of external calibration.

9. Method according to any one of the precec ing claims, in which the arterial blood pressure is sensed non-invasively using an externally mounted sensor, preferably a finger-mounted pressure sensor.

10. Method according to any one of the preceding claims, further comprising the step of generating at least one calibrated impedance value using a thermodilution method before calculating the estimate of stroke volume.

11. System for measuring cardiac output (CO) of a patient, which does not require calibration, comprising: pressure sensing means for sensing arterial blood pressure; signal processing means for converting the sensed arterial blood pressure to a pressure signal; and processing means for executing the method for measuring cardiac output (CO) of a patient according to any one of claims 1 to 10.

12. A system as in claim 11, in which the pressure sensing means is non-invasive and externally mounted on the patient preferably a finger-mounted pressure sensor.

13. Co mputer program, comprising code means adapted to perform, when operating on processing means, the method for measuring cardiac output (CO) of a patient according to any one of claims 1 to 10.

14. Computer-readable memory medium, having a program stored therein, **characterised in that** the program is the computer program according to claim 13.

Fig. A1

Fig. 1

Fig.2

Fig.3

EP 2 329 765 A1

Fig.4

Fig.5

Fig.6

Fig.7

Fig.8

Fig.9

Fig. 10

Fig.11

Fig.12

Fig. 13

Fig. 14

Fig.16

Fig. 17

Fig.18

Fig.19

Fig.20

EP 2 329 765 A1

**EUROPEAN SEARCH REPORT**

Application Number

EP 10 01 1456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 647 369 A (ROTGERS UNIVERSITY) 15 July 1997 (1997-07-15) * column 1, line 51 - line 54 * * column 2, line 60 - column 3, line 55 * * column 4, line 31 - column 13, line 12 * ----- | 1-14 | INV. A61B5/029 |
| X | US 5 400 793 A (WESSELING) 28 March 1995 (1995-03-28) * the whole document * ----- | 1-14 | |
| X | WO 99/02086 A (BAND & AL.) 21 January 1999 (1999-01-21) * claim 7 * ----- | 1-14 | |
| X | WO 99/09884 A1 (SEIKO EPSON CORP [JP]; AMANO KAZUHIKO [JP]; UEBABA KAZUO [JP]; ISHIYAM) 4 March 1999 (1999-03-04) * the whole document * ----- | 1-14 | |

TECHNICAL FIELDS SEARCHED (IPC)

A61B

The present search report has been drawn up for all claims

| Place of search | Date of completion of the search | Examiner |
|---|---|---|
| Munich | 8 March 2011 | Wetzig, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 01 1456

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2011

| Patent document cited in search report | | Publication date | Patent family member(s) | | Publication date |
|---|---|---|---|---|---|
| US 5647369 | A | 15-07-1997 | US 5535753 | A | 16-07-1996 |
| US 5400793 | A | 28-03-1995 | AT 128837 | T | 15-10-1995 |
| | | | CA 2101531 | A1 | 30-07-1992 |
| | | | DE 69205417 | D1 | 16-11-1995 |
| | | | DE 69205417 | T2 | 30-05-1996 |
| | | | DK 0569506 | T3 | 04-12-1995 |
| | | | EP 0569506 | A1 | 18-11-1993 |
| | | | ES 2080489 | T3 | 01-02-1996 |
| | | | JP 3293621 | B2 | 17-06-2002 |
| | | | JP 6505651 | T | 30-06-1994 |
| | | | NL 9100150 | A | 17-08-1992 |
| | | | WO 9212669 | A1 | 06-08-1992 |
| WO 9902086 | A | 21-01-1999 | AT 266963 | T | 15-06-2004 |
| | | | AU 729956 | B2 | 15-02-2001 |
| | | | AU 8230698 | A | 08-02-1999 |
| | | | CA 2295605 | A1 | 21-01-1999 |
| | | | DE 69824002 | D1 | 24-06-2004 |
| | | | DE 69824002 | T2 | 21-10-2004 |
| | | | DK 994672 | T3 | 27-09-2004 |
| | | | EP 0994672 | A1 | 26-04-2000 |
| | | | ES 2221988 | T3 | 16-01-2005 |
| | | | JP 2001509410 | T | 24-07-2001 |
| | | | US 6348038 | B1 | 19-02-2002 |
| WO 9909884 | A1 | 04-03-1999 | CN 1242693 | A | 26-01-2000 |
| | | | DE 69833656 | T2 | 17-08-2006 |
| | | | EP 0947160 | A1 | 06-10-1999 |
| | | | US 6361501 | B1 | 26-03-2002 |

EPO FORM P0459

For more details about this annex : see Official Journal of the European Patent Office, No. 12/82